(19) Europäisches Patentamt
European Patent Office
Office européen des brevets

(11) **EP 2 925 707 B1**

(12) **FASCICULE DE BREVET EUROPEEN**

(45) Date de publication et mention
de la délivrance du brevet:
**01.02.2017 Bulletin 2017/05**

(51) Int Cl.:
*C07C 1/24* *(2006.01)*       *C07C 11/04* *(2006.01)*
*C07C 29/76* *(2006.01)*       *C07C 31/08* *(2006.01)*
*C07C 41/09* *(2006.01)*       *C07C 43/06* *(2006.01)*

(21) Numéro de dépôt: **13803149.7**

(22) Date de dépôt: **18.11.2013**

(86) Numéro de dépôt international:
**PCT/FR2013/052768**

(87) Numéro de publication internationale:
**WO 2014/083261 (05.06.2014 Gazette 2014/23)**

(54) **PROCEDE DE DESHYDRATATION D'ETHANOL EN ETHYLENE METTANT EN OEUVRE UN PRETRAITEMENT DE LA CHARGE**

VERFAHREN ZUR DEHYDRIERUNG VON ETHANOL ZU ETHYLEN MIT VORBEHANDLUNG DES EINSATZSTOFFS

METHOD FOR DEHYDRATING ETHANOL INTO ETHYLENE, WITH PRETREATMENT OF THE FEEDSTOCK

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.11.2012 FR 1203200**

(43) Date de publication de la demande:
**07.10.2015 Bulletin 2015/41**

(73) Titulaires:
• **IFP Énergies nouvelles**
**92852 Rueil-Malmaison Cedex (FR)**
• **Total Research & Technology Feluy**
**7181 Seneffe (BE)**

(72) Inventeurs:
• **COUPARD, Vincent**
**F-69100 Villeurbanne (FR)**
• **TOUCHAIS, Natacha**
**F-38200 Vienne (FR)**

• **PLENNEVAUX, Thomas**
**F-69002 Lyon (FR)**
• **KOBEL, Emilie**
**F-69100 Villeurbanne (FR)**
• **FLEURIER, Stéphanie**
**F-69007 Lyon (FR)**
• **VEIRMEIREN, Walter**
**B-3530 Houtalen-Helchteren (BE)**
• **MINOUX, Delphine**
**B-1400 Nivelles (BE)**
• **DE SMEDT, Philip**
**B-9100 Sint-Niklaas (BE)**
• **ADAM, Cindy**
**B-5100Wierde (BE)**
• **NESTERENKO, Nikolai**
**B-1400 Nivelles (BE)**

(56) Documents cités:
**WO-A1-2010/060981       FR-A1- 2 978 145**
**US-A- 4 396 789**

**Description**

**Domaine de l'invention**

La présente invention se rapporte à un procédé de transformation de l'éthanol en éthylène et en particulier à un procédé de déshydratation de l'éthanol.

**État de la technique antérieure**

La réaction de déshydratation de l'éthanol en éthylène est connue et détaillée depuis la fin du XIXème siècle. "The Deshydration of Alcohols over Alumina. I:The reaction scheme", H. Knözinger, R. Köhne, Journal of Catalysis (1966), 5, 264-270 est considérée comme la publication de base sur les travaux de déshydratation des alcools dont l'éthanol. Il est connu que cette réaction est très endothermique, équilibrée et déplacée vers l'éthylène à haute température. La chute de température correspondant à la conversion totale de l'éthanol pur dans un réacteur adiabatique est de 380°C. À plus basse température, l'éthanol est converti en diéthyl éther (DEE). Cet "intermédiaire" de réaction peut être présent dans des procédés de déshydratation de l'éthylène dans lesquelles la conversion est partielle ou entre deux réacteurs dans des procédés multi-réacteurs. Le DEE peut ensuite être converti en éthylène à plus haute température. Le catalyseur de référence souvent utilisé est un catalyseur monofonctionnel acide, l'alumine gamma étant le catalyseur le plus cité. Les zéolithes sont également utilisées pour cette application, en particulier la ZSM5 depuis les années 1980, comme par exemple dans "Reactions of ethanol over ZSM-5",S.N. Chaudhuri & al., Journal of Molecular Catalysis 62:289-295 (1990).

Le brevet US 4,232,179 décrit un procédé de déshydratation de l'éthanol en éthylène dans lequel la chaleur nécessaire à la réaction est apportée par l'introduction dans le réacteur d'un fluide caloporteur en mélange avec la charge. Le fluide caloporteur est soit de la vapeur d'eau provenant d'une source externe, soit un flux externe provenant du procédé, soit le recycle d'une partie de l'effluent du réacteur de déshydratation, c'est à dire l'éthylène produit. L'introduction du mélange de la charge avec ledit fluide caloporteur permet de fournir la chaleur nécessaire au maintien de la température du lit catalytique à un niveau compatible avec les conversions désirées. Dans le cas où le fluide caloporteur est l'effluent du réacteur de déshydratation, un compresseur de recyclage dudit effluent est nécessaire. Cependant, le recyclage de l'éthylène produit par la réaction est un inconvénient car l'introduction de l'éthylène modifie l'équilibre de la réaction de déshydratation. De plus, l'éthylène participe aux réactions secondaires d'oligomérisation, de transfert d'hydrogène et de disproportionation des oléfines qui sont des réactions d'ordre supérieur à 0 par rapport à leur réactif. L'augmentation de la concentration en éthylène dès le début de la réaction multiplie la formation de produits secondaires. La perte en éthylène est donc plus importante, ce qui se traduit par une baisse de la sélectivité.

La demande de brevet WO 2007/134415 décrit un procédé de déshydratation de l'éthanol en éthylène amélioré par rapport à celui du brevet US 4 232 179 permettant un coût d'investissement réduit, grâce à un nombre réduit d'équipements et un coût opérationnel réduit, grâce à la non utilisation de vapeur d'eau externe au procédé. Dans ce procédé, au moins une partie de l'effluent du réacteur de déshydratation (mélange d'éthylène produit et de vapeur d'eau) et de la vapeur d'eau surchauffée obtenue à partir de l'eau produite par la déshydratation de l'éthanol et condensée dans le réacteur, sont utilisés comme fluide caloporteur et entrent dans le réacteur de déshydratation en mélange avec l'éthanol. Ladite demande de brevet est muette sur la condition de pression à respecter entre la charge éthanol et l'effluent dans le but de maximiser l'échange de chaleur.

Le brevet US 4,396,789 décrit également un procédé de déshydratation de l'éthanol en éthylène dans lequel l'éthanol et la vapeur d'eau agissant comme fluide caloporteur sont introduits dans le premier réacteur à une température comprise entre 400 et 520°C et à une pression élevée comprise entre 2 et 4 MPa (20 et 40 atm), de sorte que l'effluent produit par la réaction de déshydratation est soutiré du dernier réacteur à une pression au moins supérieure à 1,8 MPa (18 atm), ledit produit de réaction, c'est à dire l'éthylène, pouvant subir après refroidissement l'étape de distillation cryogénique finale sans étape de compression intermédiaire. Ledit procédé se caractérise également par un échange de chaleur entre ledit produit de la réaction de déshydratation et la charge introduite dans le premier réacteur, ledit produit de réaction étant utilisé pour vaporiser la charge entrant dans le premier réacteur. L'éthanol non converti, au moins une partie de l'eau formée au cours des réactions du procédé et l'eau ajoutée pour le lavage final des gaz sont recyclés pour assurer la conversion complète de l'éthanol.

La demande de brevet WO 2011/002699 divulgue un procédé de déshydratation d'une charge éthanol en éthylène comprenant la vaporisation d'un mélange d'éthanol et d'eau et la réaction de ce mélange dans un réacteur adiabatique. Cette demande décrit la possibilité de convertir l'éthanol en diethylether dans le premier réacteur de déshydratation dans une gamme de température allant de 200 à 450°C. Il ne mentionne ni l'utilisation de résine dans ce premier réacteur, ni l'augmentation de l'activité du catalyseur des réacteurs suivant du a la séparation des poisons. De plus, cette demande n'adresse pas le problème de la maximisation de la récupération de chaleur en vue de réduire la consommation énergétique du procédé.

Le brevet WO 2010/060981 décrit une solution de prétraitement d'une charge éthanol éventuellement biosourcée avant son passage dans un réacteur contenant un catalyseur acide. Il peut en particulier s'appliquer a la déshydratation de l'éthanol en éthylène. Le but du prétraitement décrit dans ce brevet est de limiter l'inhibition ou l'empoisonnement du catalyseur par des composants contenus dans la charge éthanol. Le prétraitement peut se faire grâce a un absorbant ou grâce a un catalyseur d'hydrogénation permettant de convertir au moins une partie des composant nocifs pour le catalyseur en composants neutres. Ce brevet ne décrit pas la possibilité d'utiliser un catalyseur ou une résine dans le prétraitement conduisant à la déshydratation de l'éthanol en diethylether.

Le brevet JP11043452A décrit une solution de prétraitement d'une charge éthanol mettant en oeuvre une résine échangeuse d'ion, du kaolin ou une zéolithe pour réduire la quantité de composé azoté basiques en dessous de 10 ppm poids. Il ne mentionne pas la formation ou l'effet du diethylether sur la vaporisation de charge.

Un objectif de l'invention est de fournir un procédé de déshydratation de l'éthanol en éthylène comprenant une étape de prétraitement de la charge éthanol à l'aide d'un solide acide afin de limiter la quantité d'azote organique, qui réduit la durée de vie du catalyseur, et de convertir partiellement l'éthanol en DEE.

## Résumé et intérêt de l'invention

L'invention décrit un procédé de déshydratation d'une charge éthanol en éthylène comprenant une étape de prétraitement. La dite étape de prétraitement réduit le taux d'azote organique ou basique contenu dans ladite charge et convertit une fraction de l'éthanol en DEE.

Ladite invention présente l'avantage par rapport aux procédés de l'art antérieur d'augmenter le temps de cycle du catalyseur de déshydratation de l'éthanol en piégeant les impuretés cationiques ou anioniques, les impuretés basiques, complexantes, chélatantes, les impuretés inorganiques ou organiques comme par exemple, l'azote présent dans la charge sous forme basique, par exemple sous forme d'ammoniaque et/ou espèces organiques et basiques, par exemple sous forme d'amine, amide, imine ou nitrile lors de l'étape de prétraitement. Le piégeage des composés azotés a en particulier pour effet d'améliorer l'activité des catalyseurs acides utilisés en déshydratation.

Dans un arrangement préféré comprenant une compression de la charge vaporisée, la présente invention présente l'avantage, par rapport aux procédés de l'art antérieur, de maximiser l'échange de chaleur entre la charge et l'effluent issu du dernier réacteur de déshydratation, c'est à dire d'échanger la totalité de l'enthalpie de vaporisation de la charge et la majeure partie de l'enthalpie de condensation dudit effluent grâce à l'introduction de la charge dans l'étape a) de vaporisation à une pression inférieure à la pression de l'effluent en sortie du dernier réacteur.

La demanderesse a découvert que, de manière surprenante, ladite étape de prétraitement menée dans les conditions opératoires selon l'invention aboutissait à la conversion partielle d'éthanol en DEE et permettait de diminuer significativement la consommation énergétique de la production d'éthylène.

## Description de l'invention

L'invention concerne un procédé de déshydratation d'une charge éthanol en éthylène comprenant au moins les étapes :

a) Une étape de prétraitement de la charge éthanol sur un solide acide opérant à une température comprise entre 100 et 130°C de manière à produire une charge éthanol prétraitée,

b) Une étape de vaporisation d'une charge de vaporisation comprenant ladite charge éthanol prétraitée dans un échangeur de chaleur, ladite charge de vaporisation étant introduite dans ladite étape de vaporisation à une pression comprise entre 0,1 et 2,5 MPa de manière à produire une charge vaporisée,

c) Une étape de surchauffe de ladite charge vaporisée de manière à l'amener à une température d'entrée compatible avec la température de la réaction de déshydratation,

d) Une étape de déshydratation de ladite charge issue de l'étape c) dans au moins un réacteur adiabatique contenant au moins un catalyseur de déshydratation et dans lequel la réaction de déshydratation a lieu, opérant à une température d'entrée comprise entre 350 et 550 °C et à une pression d'entrée comprise entre 0,3 et 1,8 MPa.

## Charge

Conformément à l'invention, la charge traitée dans le procédé de déshydratation est une charge éthanol.

Ladite charge éthanol est avantageusement une charge éthanol concentrée. On entend par charge éthanol concentrée une charge éthanol comprenant un pourcentage massique en éthanol supérieur ou égal à 35% poids. De préférence, ladite charge éthanol concentrée comprend un pourcentage massique en éthanol compris entre 35 et 99,9% poids.

La charge éthanol comprenant moins de 35% poids d'éthanol peut être concentrée par tous moyens connus de l'Homme du métier, par exemple par distillation, par absorption, par pervaporation.

Ladite charge éthanol comprend également avantageusement, en plus de l'eau, une teneur en alcools autres que l'éthanol, tels que par exemple le méthanol, le butanol et/ou l'isopentanol inférieure à 10 % poids, et de préférence inférieure à 5 % poids, une teneur en composés oxygénés autres que les alcools tels que par exemple les éthers, les acides, les cétones, les aldéhydes et/ou les esters inférieure à 1 % poids et une teneur en azote et en soufre, organique et minéral, inférieure à 0,5 % poids, les pourcentages poids étant exprimés par rapport à la masse totale de ladite charge.

La charge éthanol traitée dans le procédé selon l'invention est éventuellement obtenue par un procédé de synthèse d'alcool à partir de ressources fossiles telles que par exemple à partir du charbon, du gaz naturel ou des déchets carbonés.

Ladite charge peut également avantageusement provenir de ressources non fossiles. De préférence, la charge éthanol traitée dans le procédé selon l'invention est une charge éthanol produite à partir de source renouvelable issue de la biomasse, souvent appelée "bioéthanol". Le bioéthanol est une charge produite par voie biologique, de préférence par fermentation de sucres issus par exemple des cultures de plantes sucrières comme la canne à sucre (saccharose, glucose, fructose, et sucrose), des betteraves, ou encore des plantes amylacées (amidon) ou de la biomasse lignocellulosique ou de cellulose hydrolysée (glucose majoritaire et xylose, galactose), contenant des quantités variables d'eau.

Pour une description plus complète des procédés fermentaires classiques, on peut se reporter a l'ouvrage 'Les Biocarburants, État des lieux, perspectives et enjeux du développement, Daniel Ballerini, Editions Technip'.

Ladite charge peut également avantageusement être obtenue à partir de gaz de synthèse.

Ladite charge peut également avantageusement également être obtenue par hydrogénation des acides ou esters correspondants. Dans ce cas, l'acide acétique ou les esters acétiques sont avantageusement hydrogénés à l'aide d'hydrogène en éthanol. L'acide acétique peut avantageusement être obtenu par carbonylation du méthanol ou par fermentation des carbohydrates.

De préférence, la charge éthanol traitée dans le procédé selon l'invention est une charge éthanol produite à partir de source renouvelable issue de la biomasse.

**Étape a) de prétraitement**

Conformément à l'invention, la charge éthanol subit une étape a) de prétraitement de manière à produire une charge éthanol prétraitée. Ladite étape de prétraitement permet d'éliminer les composés azotés présents dans ladite charge de manière à limiter la désactivation du catalyseur de déshydratation placé en aval.

Ladite étape a) de prétraitement est mise en oeuvre sur un solide acide, de préférence une résine acide, et à une température comprise entre 100 et 130 °C, préférentiellement entre 110°C et 130°C.

La charge éthanol est avantageusement chauffée à la température de l'étape a) de prétraitement dans un échangeur de chaleur, grâce à un échange de chaleur avec une source de chaleur externe au procédé, par exemple par chauffage direct (par exemple dans un four) ou tout autre technique connue de l'homme du métier.

Ladite étape a) de prétraitement permet d'éliminer les impuretés, basiques et/ou organiques, et les espèces cationiques afin d'obtenir une charge éthanol prétraitée répondant au niveau d'impuretés compatibles avec le catalyseur de déshydratation.

Le prétraitement sur le solide acide dans les conditions opératoires selon l'invention permet de convertir entre 3% poids et 20% poids, préférentiellement entre 8 et 12% poids de l'éthanol présent dans ladite charge en DEE, le pourcentage poids étant déterminé par rapport au poids total d'éthanol présent dans ladite charge en entrée de l'étape a) de prétraitement.

Le solide acide comprend tout les solides acides connus par l'Homme de l'art : des silice-alumines, des argiles acides, des zéolithes, des zircones sulfatées, des résines acides, etc. L'essentiel est que le solide acide possède une capacité d'échange élevée pour capter le plus possible les espèces basiques et cationiques et une force d'acidité suffisamment élevée pour effectuer la transformation partielle de l'éthanol en DEE.

Des solides acides qui sont communément disponibles commercialement sont les argiles traitées aux acides pour les rendre acides (comme la montmorillonite) et les zéolithes, ayant un rapport silice sur alumine dans le réseau cristallin de 2.5 à 100 molaire. Les résines acides comprennent des groupes sulfoniques, greffées sur un support organique composé de chaines aromatiques et/ou haloaliphatiques. De préférence les solides acides possèdent une capacité d'échange d'au moins de 0,1 mmol $H^+$ équivalent par gramme.

La résine acide comprend des groupes sulfoniques acides et est préparée par polymérisation ou copolymérisation de groupe vinyliques aromatiques suivie d'une sulfonation, lesdits groupes vinyliques aromatiques étant choisis parmi le styrène, le vinyle toluène, le vinyle naphtalène, le vinyle éthyle benzène, le méthyle styrène, le vinyle chlorobenzene et le vinyle xylène, ladite résine présentant un taux de réticulation compris entre 20 et 35 %, de préférence entre 25 et 35 % et de manière préférée, égale à 30 % et une force acide dosée par potentiométrie lors de la neutralisation par une solution de KOH, de 0,2 à 6 mmol $H^+$ équivalent par gramme et de préférence entre 0,2 et 2,5 mmol $H^+$ équivalent par gramme.

Ladite résine échangeuse d'ions acide contient entre 1 et 2 groupes sulfoniques terminaux par groupe aromatique. Sa taille est comprise entre 0,15 et 1,5 mm. Par taille de la résine on entend le diamètre de la plus petite sphère englobant la particule de résine. On mesure les classes de taille de résine par tamisages sur des tamis adaptés selon une technique connue de l'homme du métier.

Une résine préférée est une résine constituée par des co-polymères de monovinyle aromatiques et polyvinyle aromatiques, et de manière très préférée, de copolymère de di-vinyle benzène et de polystyrène présentant un taux de réticulation compris entre 20 et 45 %, de préférence entre 30 et 40 %, et de manière préférée égale à 35 % et une force acide, représentant le nombre de sites actifs de ladite résine, dosée par potentiométrie lors de la neutralisation par une solution de KOH, compris entre 1 et 10 mmol $H^+$ équivalent par gramme et de préférence compris entre 3,5 et 6 mmol $H^+$ équivalent par gramme. Par exemple, la résine est une résine TA801 vendue par société Axens.

Les solides acides peuvent être régénérés de temps en temps une fois que la capacité d'échange est presque saturée par l'adsorption des espèces basiques et cationiques in situ ou ex situ. Dans le cas des solides acides inorganiques comme les argiles et zéolithes, la régénération peut consister en un simple chauffage à haute température afin de désorber les espèces basiques en présence d'un flux inerte ou contenant de l'oxygène. Les cations peuvent être enlevés par échange ionique. Les résines acides peuvent être régénérées par échange ionique, typiquement par un traitement avec un acide en phase liquide. Les solides acides peuvent également être utilisés une fois jusqu'à saturation et remplacés par du solide vierge.

Le solide acide peut être utilisé seul ou en mélange avec d'autres types de solides acide. Des mélanges de différents solides acides ou des séquences de solides acides peuvent être mises en oeuvre afin d'optimiser la capacité d'adsorber les espèces basiques et cationiques et la capacité de transformer partiellement l'éthanol en DEE.

Le prétraitement décrit ci-dessus peut avantageusement être complété par un prétraitement utilisant une résine échangeuse d'anion. Cette résine peut par exemple être une résine chargée en sodium, ou triméthylamonium caractérisée par une capacité d'échange mesurée en mg(OH⁻)/litre. Cette résine peut par exemple être de la résine Amberlite IRN78. Cette résine supplémentaire permet de retenir les ions sulfates $SO_4^{2-}$ afin de prolonger la vie du catalyseur.

**Étape b) de vaporisation**

On appelle charge de vaporisation la charge comprenant ladite charge éthanol prétraitée. Ladite charge de vaporisation comprend également avantageusement un flux d'eau recyclée selon l'étape e) de recyclage ou un flux d'eau externe au procédé. Dans ce cas, le rapport massique du flux d'eau, qu'il soit recyclé ou externe au procédé, sur le flux éthanol prétraité est avantageusement compris entre 1 et 4, dans le but d'abaisser les pressions partielles d'éthanol dans le ou les réacteur de déshydratation et de rendre le procédé plus sélectif en éthylène.

Conformément à l'invention, le procédé de déshydratation comprend une étape b) de vaporisation de ladite charge de vaporisation de manière à produire une charge vaporisée. Ladite vaporisation est réalisée grâce à un échange de chaleur dans un échangeur de chaleur avec une source de chaleur qui peut être un flux interne ou externe au procédé, ou par chauffage direct (par exemple dans un four) ou tout autre technique connue de l'homme du métier.

De manière surprenante, à pression donnée, la température de vaporisation de la charge de vaporisation est abaissée par rapport à celle d'une charge obtenue par un enchaînement qui ne comprendrait pas l'étape a) de prétraitement.

Ladite charge de vaporisation est introduite dans ladite étape de b) de vaporisation à une pression comprise entre 0,1 et 2,5 MPa.

**Étape optionnelle de compression**

Dans un arrangement préféré, ladite charge vaporisée, subit une compression dans une étape de compression de manière à produire une charge comprimée. Ladite étape de compression est avantageusement mise en oeuvre dans tout type de compresseur connu de l'homme du métier. En particulier, l'étape de compression est avantageusement mise en oeuvre dans un compresseur de type compresseur radial à multiplicateur intégré ou dans un compresseur comprenant une ou plusieurs soufflantes avec une roue radiale mise en série sans refroidissement intermédiaire ou dans un compresseur de type volumétrique avec ou sans lubrification.

L'étape optionnelle de compression permet de réaliser une pompe à chaleur intégrée audit procédé, utilisant les flux issus du procédé, en permettant de vaporiser la charge de vaporisation de l'étape b) par échange de chaleur avec l'effluent issu de l'étape d) de déshydratation.

Dans le cas où l'on effectue l'étape optionnelle de compression, ladite charge de vaporisation est introduite dans ladite étape b) de vaporisation à une pression comprise entre 0,1 et 1,4 MPa, préférentiellement entre 0,2 et 0,6 MPa.

La pression de ladite charge comprimée à l'issue de l'étape optionnelle de compression est avantageusement comprise entre 0,3 et 1,8 MPa, préférentiellement entre 0,5 et 1,3 MPa. La pression de sortie de ladite charge est suffisamment élevée pour que la température de condensation de l'effluent issu du dernier réacteur soit supérieure à la température de vaporisation de la charge entrant dans l'étape b), ce qui est une condition nécessaire à la faisabilité de l'étape b).

De manière surprenante, à pression donnée, la température de vaporisation de la charge de vaporisation est abaissée par rapport à celle d'une charge obtenue par un enchaînement qui ne comprendrait pas l'étape a) de prétraitement. Pour une température de condensation de l'effluent de l'étape d) de déshydratation donnée et une approche thermique fixée dans l'échangeur de chaleur permettant l'échange de chaleur entre ladite charge de vaporisation et ledit effluent de l'étape d), on peut donc ajuster la pression en amont de l'étape b) de vaporisation à une valeur plus élevée que ce qu'elle aurait été dans un enchaînement ne comprenant pas l'étape a) de prétraitement. Le taux de compression nécessaire dans l'étape optionnelle de compression est ainsi réduit pour atteindre une pression donnée à l'issue de ladite étape, réduisant la consommation énergétique de ladite étape.

La combinaison des conditions opératoires spécifiques de l'étape b) et de l'étape optionnelle de compression permet d'éviter l'apport de fluide caloporteur externe au procédé pour assurer la vaporisation de ladite charge de vaporisation en récupérant la majeure partie de la chaleur latente de la phase aqueuse de l'effluent issu de l'étape d) pour vaporiser la charge de vaporisation. Ainsi, seuls les flux issus du procédé sont utilisés

**Étape c) de surchauffe**

Ladite charge vaporisée, éventuellement comprimée est chauffée dans un échangeur de type monophasique gaz, grâce à un échange de chaleur avec tout flux interne ou externe au procédé, de préférence grâce à un échange de chaleur avec l'effluent issu du dernier réacteur adiabatique de l'étape d). Dans ledit échangeur de type monophasique gaz, ladite charge, éventuellement comprimée, est surchauffée. Dans le cas où l'échange de chaleur est réalisé avec l'effluent issu, à l'état gazeux, du dernier réacteur adiabatique de l'étape d), ce dernier est "désurchauffé" sans être condensé.

Dans le cas où l'on réalise l'étape optionnelle de compression, ledit échangeur de type monophasique gaz est un échangeur d'une technologie connue de l'Homme du métier qui permet de minimiser les pertes de charge tout en ayant une grande surface d'échange. Cet échange gaz/gaz à basse pression induit une densité de flux de chaleur faible à travers la paroi de l'échangeur (coefficient de transfert faible), ce qui force à avoir une grande surface d'échange. De plus, la perte de pression doit etre minimisée afin de limiter la charge du compresseur de l'étape optionnelle de compression. Par exemple cet échangeur peut être un échangeur à plaques pressurisées dans une calandre, de type Packinox fourni par Alphalaval.

Ladite charge vaporisée, éventuellement comprimée, éventuellement chauffée dans ledit échangeur de type monophasique gaz, est ensuite introduite dans un équipement de surchauffe, de préférence un four, de manière à l'amener à une température d'entrée dans au moins un réacteur adiabatique compatible avec la température de la réaction de déshydratation.

La présence de l'étape a) de prétraitement permet d'augmenter l'activité du catalyseur et de diminuer la température d'entrée de la charge dans l'étape d) de déshydratation.

**Étape d) de déshydratation**

Conformément à l'invention, ladite charge issue de l'étape c) subit une étape d) de déshydratation dans au moins un réacteur adiabatique contenant au moins un lit fixe de catalyseur de déshydratation et dans lequel la réaction de déshydratation a lieu.

L'étape d) de déshydratation est avantageusement réalisée dans un ou deux réacteurs.

Dans le cas où l'étape d) est mise en oeuvre dans un réacteur adiabatique, ladite charge comprimée, et éventuellement chauffée, est avantageusement introduite dans ledit réacteur à une température d'entrée comprise entre 350 et 550 °C et de préférence entre 400 et 500 °C, et à une pression d'entrée comprise entre 0,3 et 1,8 MPa, et de préférence entre 0,4 et 0,8 MPa.

L'effluent issu dudit réacteur adiabatique de l'étape d) présente avantageusement une température comprise entre 270 et 450 °C et de préférence entre 340 et 430 °C, et une pression de sortie comprise entre 0,2 et 1,6 MPa et de preference entre 0,3 et 0,8 MPa.

Dans le cas où l'étape d) est mise en oeuvre dans deux réacteurs adiabatiques, ladite charge comprimée, et éventuellement chauffée, est avantageusement introduite dans le premier réacteur à une température d'entrée comprise entre 350 et 550 °C et de préférence à une température comprise entre 370 et 500 °C, et à une pression d'entrée comprise entre 0,3 et 1,8 MPa, et de préférence entre 0,4 et 1,1 MPa.

L'effluent issu du premier réacteur adiabatique sort avantageusement dudit premier réacteur à une température comprise entre 270 et 450 °C et de préférence entre 290 et 390 °C, et à une pression comprise entre 0,3 et 1,7 MPa et de préférence entre 0,3 et 1,0 MPa.

Ledit effluent est ensuite avantageusement introduit dans un four de manière à ce que la température d'entrée dudit effluent dans le deuxième réacteur adiabatique soit comprise entre 350 et 550 °C et de préférence entre 400 et 500 °C. Ledit effluent présente une pression d'entrée dans ledit deuxième réacteur avantageusement comprise entre 0,3 et 1,7 MPa et de préférence entre 0,3 et 0,9 MPa.

L'effluent issu du deuxième réacteur adiabatique sort dudit deuxième réacteur adiabatique à une température avantageusement comprise entre 270 et 450 °C et de préférence entre 340 et 430 °C. La pression de sortie dudit effluent issu du deuxième réacteur adiabatique est avantageusement comprise entre 0,2 et 1,6 MPa et de préférence entre 0,3 et 0,8 MPa.

La température d'entrée du ou des réacteurs peut avantageusement être graduellement augmentée pour éviter la désactivation du catalyseur de déshydratation.

La réaction de déshydratation qui a lieu dans au moins un réacteur adiabatique de l'étape d) du procédé selon l'invention opère avantageusement à une vitesse pondérale horaire comprise entre 0,1 et 20 $h^{-1}$ et de préférence entre 0,5 et 15 $h^{-1}$. La vitesse pondérale horaire est définie comme étant le rapport du débit massique de la charge éthanol pure sur la masse de catalyseur.

Le catalyseur de déshydratation utilisé dans l'étape d) est un catalyseur connu de l'Homme du métier. Ledit catalyseur est un catalyseur acide amorphe, un catalyseur acide zéolithique, un catalyseur à base silice-alumine, un catalyseur a base d'alumine ou un catalyseur a base d'alumine silicée.

Ledit catalyseur est de préférence un catalyseur acide amorphe ou un catalyseur acide zéolithique.

Dans le cas où le catalyseur de déshydratation utilisé dans l'étape d) est un catalyseur zéolithique, ledit catalyseur comprend au moins une zéolithe choisie parmi les zéolithes ayant au moins des ouvertures de pores contenant 8, 10 ou 12 atomes d'oxygène (8 MR, 10 MR ou 12 MR). Il est connu en effet de définir la taille des pores des zéolithes par le nombre d'atomes d'oxygène formant la section annulaire des canaux des zéolithes, appelés "member ring" ou MR en anglais. De manière préférée, ledit catalyseur de déshydratation zéolithique comprend au moins une zéolithe présentant un type structural choisi parmi les types structuraux MFI, FAU, MOR, FER, SAPO, TON, CHA, EUO, MEL et BEA. De préférence, ledit catalyseur de déshydratation zéolithique comprend une zéolithe de type structural MFI et de manière préférée une zéolithe ZSM-5.

La zéolithe mise en oeuvre dans le catalyseur de déshydratation utilisé dans l'étape d) du procédé selon l'invention peut avantageusement être modifiée par désalumination ou désilication selon toute méthode de désalumination ou désilication connue de l'homme du métier.

La zéolithe mise en oeuvre dans le catalyseur de déshydratation utilisé dans l'étape d) du procédé selon l'invention ou le catalyseur final peut avantageusement être modifiée par un agent de nature à atténuer son acidité totale et à améliorer ses propriétés de résistance hydrothermales. De préférence, ladite zéolithe ou ledit catalyseur comprend avantageusement du phosphore, de préférence ajouté sous forme $H_3PO_4$ suivi d'un traitement à la vapeur après neutralisation de l'excès d'acide par un précurseur basique tels que par exemple le calcium Ca. De manière préférée, ladite zéolithe comprend une teneur en phosphore comprise entre 1 et 4.5%poids, de préférence entre 1.5 et 3.1%poids poids par rapport à la masse totale du catalyseur.

De préférence, le catalyseur de déshydratation utilisé dans l'étape d) du procédé selon l'invention est le catalyseur décrit dans les demandes de brevet WO/2009/098262 WO/2009/098267, WO/2009/098268, ou WO/2009/098269.

Dans le cas où le catalyseur de déshydratation utilisé dans l'étape d) est un catalyseur acide amorphe, ledit catalyseur comprend au moins un oxyde réfractaire poreux choisi parmi l'alumine, l'alumine activée par un dépôt d'acide minéral et la silice alumine.

Ledit catalyseur de déshydratation amorphe ou zéolithique utilisé dans l'étape d) du procédé selon l'invention peut avantageusement également comprendre au moins une matrice de type oxyde également appelé liant. On entend par matrice selon l'invention, une matrice amorphe, cristallisée, ou comprenant des parties amorphes et cristallisées. Ladite matrice est avantageusement choisie parmi les éléments du groupe formé par les argiles (telles que par exemple parmi les argiles naturelles telles que le kaolin ou la bentonite), la magnésie, les alumines, les silices, les silice-alumines, les aluminates, l'oxyde de titane, l'oxyde de bore, la zircone, les phosphates d'aluminium, les phosphates de titane, les phosphates de zirconium, et le charbon, utilisés seuls ou en mélange. De préférence, ladite matrice est choisie parmi les éléments du groupe formé par les alumines, les silices et les argiles.

Ledit catalyseur de déshydratation utilisé dans l'étape d) du procédé selon l'invention est avantageusement mis en forme sous la forme de grains de différentes formes et dimensions. Il est avantageusement utilisé sous la forme d'extrudés cylindriques ou polylobés tels que bilobés, trilobés, polylobés de forme droite ou torsadée, mais peut éventuellement être fabriqué et employé sous la forme de poudre concassée, de tablettes, d'anneaux, de billes, de roues, de sphères. De préférence, ledit catalyseur est sous forme d'extrudés.

Ledit catalyseur de déshydratation utilisé dans l'étape d) du procédé selon l'invention est avantageusement mis en oeuvre dans au moins un réacteur, en lit fixe ou en lit mobile.

Dans l'étape d) du procédé selon l'invention, les catalyseurs utilisés et les conditions opératoires sont choisis de manière à maximiser la production d'éthylène. Les réactions globales de déshydratation mises en oeuvre dans l'étape d) du procédé selon l'invention sont les suivantes :

$$2\ C_2H_5OH \rightarrow 2\ CH_2=CH_2 + 2\ H_2O$$

$$CH_3CH_2OCH_2CH_3 \rightarrow 2\ CH_2=CH_2 + H_2O$$

La conversion de la charge éthanol dans l'étape d) est supérieure à 90 %, de préférence 95 % et de manière préférée supérieure à 99 %.

Une conversion inférieure à 90% a pour effet de baisser le rendement global du procédé, une quantité plus importante de DEE non converti en éthylène étant perdue dans les étapes de séparation avale. La conversion de la charge éthanol est définie, en pourcentage, par la formule suivante :

[1 − (masse horaire d'éthanol en sortie/masse horaire d'éthanol en entrée)]x100.

La masse horaire d'éthanol en entrée et en sortie est mesurée de manière classique par exemple par chromatographie.

L'étape d) dans laquelle la réaction de déshydratation a lieu est avantageusement réalisée dans un ou deux réacteurs. Un réacteur préféré est un réacteur radial fonctionnant en mode ascendant ou descendant. Lors de l'étape d) du procédé selon l'invention, la transformation de la charge s'accompagne de la désactivation du catalyseur de déshydratation par cokage et/ou par adsorption de composés inhibiteurs. Le catalyseur de déshydratation doit donc subir périodiquement une étape de régénération. De préférence, le réacteur est utilisé dans un mode de régénération alterné, aussi appelé réacteur swing, afin d'alterner les phases de réaction et de régénération dudit catalyseur de déshydratation. L'objectif de ce traitement de régénération est de brûler les dépôts organiques ainsi que les espèces contenant de l'azote et du soufre, contenues à la surface et au sein dudit catalyseur de déshydratation. L'étape a) de prétraitement mise en oeuvre dans cette invention permet de réduire la quantité d'impuretés, basiques et organiques, ainsi que les espèces cationiques qui vont venir altérer la durée de cycle du catalyseur. L'élimination de ces espèces permet ainsi de limiter le nombre de régénération du catalyseur.

Optionnellement, par exemple dans le cas ou la charge de vaporisation ne comprend pas de flux d'eau recyclée ou de flux d'eau externe au procédé, le nombre de réacteurs peut être augmenté afin de compenser l'endothermicité de la réaction par la présence de fours intermédiaires dans l'avancement de la réaction de déshydratation de l'éthanol.

La régénération du catalyseur de déshydratation utilisé dans ladite étape d) est avantageusement réalisée par oxydation du coke et des composés inhibiteurs sous flux d'air ou sous un mélange air/azote, par exemple en utilisant une recirculation de l'air de combustion avec ou sans eau afin de diluer l'oxygène et maîtriser l'exotherme de régénération. Dans ce cas, on peut avantageusement ajuster la teneur en oxygène en entrée du réacteur par un appoint d'air. La régénération a lieu à pression comprise entre la pression atmosphérique et la pression de réaction.

La température de régénération est avantageusement choisie entre 400 et 600 °C ; elle peut avantageusement varier en cours de régénération. La fin de la régénération est détectée quand il n'y a plus de consommation d'oxygène, signe d'une combustion totale du coke.

L'effluent issu du dernier réacteur adiabatique de l'étape d) est éventuellement envoyé dans un échangeur de type monophasique gaz dans lequel il est "désurchauffé" sans être condensé par échange de chaleur avec la charge comprimée issue de l'étape optionnelle de compression, qui elle est surchauffée.

Ledit effluent "désurchauffé" est ensuite avantageusement envoyé dans un deuxième échangeur de type gaz/liquide dans lequel il est condensé partiellement par un échange de chaleur servant à vaporiser la charge de vaporisation.

Ledit effluent est ensuite préférentiellement encore refroidi par échange de chaleur avec la charge éthanol lors de la préchauffe de la charge éthanol.

**Étape e) de purification et recyclage**

Optionnellement, l'effluent issu de l'étape d) de déshydratation subi ensuite un refroidissement, par exemple dans une colonne de séparation gaz/liquide.

De préférence, au moins une partie du flux d'eau traitée issu de la condensation de l'effluent est recyclée en amont de l'étape b) de vaporisation.

L'effluent issu de l'étape d) subi ensuite de préférence une étape de compression et une étape de purification selon des moyens connus de l'Homme du métier. De préférence, ces étapes permettent de séparer les composants qui détériorent la polymérisation de l'éthylène, en particulier l'acétaldéhyde et le $CO_2$.

**Description des figures**

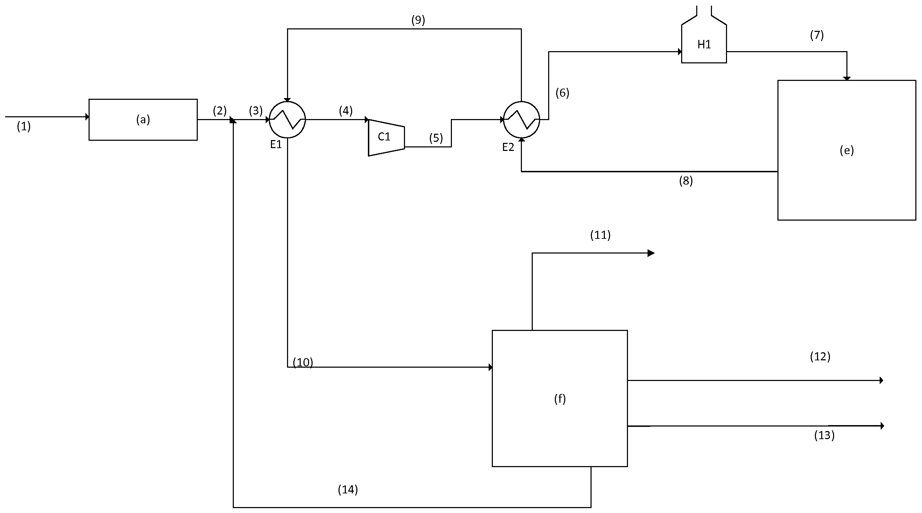

La figure 1 représente schématiquement un arrangement particulier du procédé de déshydratation d'une charge d'éthanol mettant en oeuvre un prétraitement avec un recyclage d'au moins une partie de l'eau traitée lors de l'étape (e) du procédé.

La charge éthanol (1) est introduite dans une zone de prétraitement (a). La charge éthanol prétraitée (2) est ensuite mélangée dans la conduite (3) avec une partie du flux d'eau traitée issu de la zone de purification (f) qui est recyclée de manière à servir de diluant réactionnel via la conduite (14). Ce mélange, constituant la charge de vaporisation, est introduit via la conduite (3) dans un échangeur gaz/liquide E1 dans lequel ledit mélange subit un échange de chaleur avec l'effluent issu de la section de déshydratation (e) qui pénètre dans l'échangeur via la conduite (9) de manière à produire une charge vaporisée. La chaleur latente, dite également enthalpie de condensation, de l'effluent issu de la zone de déshydratation (e) est utilisée pour vaporiser la charge de vaporisation, sans apport de chaleur externe.

La charge vaporisée est ensuite envoyée via la conduite (4) dans un compresseur C1.

Ladite charge vaporisée et comprimée est ensuite envoyée via la conduite (5) dans un échangeur E2 de type monophasique gaz, dans lequel ladite charge est chauffée grâce à un échange de chaleur avec l'effluent issu de la section de déshydratation (e) qui est introduit dans E2 via la conduite (8). Dans ledit échangeur de type monophasique gaz, ladite charge vaporisée et comprimée est surchauffée et l'effluent issu, à l'état gazeux, de la section de déshydratation (e) est "désurchauffé", sans être condensé.

Ladite charge vaporisée, comprimée et chauffée dans l'échangeur de type monophasique gaz E2 est ensuite introduite dans un four H1 via la conduite (6) de manière à l'amener à une température d'entrée dans la section de déshydratation (e) compatible avec la température de la réaction de déshydratation.

L'effluent issu de la section de déshydratation (e) subit ensuite les deux échanges successifs décrits précédemment dans les échangeurs E2 et E1 via les conduites (8) et (9).

L'effluent issu de l'échangeur E1 est envoyé via la conduite (10) dans la section de purification (f) où il est séparé en au moins un effluent comprenant de l'éthylène (12), au moins une purge comprenant de l'eau (13), au moins un effluent comprenant de l'eau et la totalité (optionnellement une partie) de l'éthanol non réagit (14), et au moins un effluent comprenant les gaz légers (11).

Les exemples suivants illustrent l'invention sans en limiter la portée.

**Exemples**

**Exemple 1 : conforme à l'invention**

L'exemple 1 illustre un procédé selon l'invention.

La charge éthanol considérée est produite par fermentation de blé, sans extraction des glutens, par un procédé de type dry milling selon le terme anglo-saxon.

Étape a)

La charge éthanol est introduite, à un débit de 45 664 kg/h, à une température de 120°C et une pression de 1.15 MPa sur une résine de prétraitement TA801 afin d'éliminer les traces de composés azotés. Lors de ce prétraitement, une partie de l'éthanol est convertie en DEE. Les caractéristiques de la charge éthanol brut et prétraitée sont données dans le Tableau 1.

Tableau 1 : Caractéristiques de la charge éthanol avant et après prétraitement (pourcentages massiques)

| | CHARGE ETHANOL | ETHANOL APRES PRETRAITEMENT |
|---|---|---|
| ETHANOL | 91,2% | 82,1% |
| H2O | 8,7% | 10.5% |
| DEE | 0% | 7,3% |
| COMPOSES AZOTE | 0,005% | 0,000% |

Étape b)

La charge de vaporisation, constituée de la charge éthanol prétraitée en mélange avec 141 252 kg/h d'eau traitée et d'éthanol non converti recyclés selon l'étape (f), est dépressurisée et introduite dans un échangeur E1 à une pression égale à 0,27 MPa. La température de bulle de cette charge à cette pression est de 127 °C compte tenu de la présence de DEE. La charge de vaporisation entre dans l'échangeur E1 à 113 °C et est donc déjà vaporisé à 8,6 % massique. La pression en entrée de l'échangeur E1 a été ajustée de telle manière que l'approche thermique avec le flux issu du dernier réacteur adiabatique de l'étape e) soit au minimum de 15 °C.

Dans l'étape b), la majorité de la chaleur latente de la phase aqueuse de l'effluent issu du dernier réacteur adiabatique de l'étape e) est récupérée pour vaporiser la charge de vaporisation, sans apport de chaleur externe. Ainsi, 93,6 MW sont échangés entre ladite charge de vaporisation et ledit effluent.

Étape optionnelle de compression

La charge vaporisée est ensuite comprimée dans l'étape optionnelle de compression grâce à un compresseur radial à multiplicateur intégré de manière à ce que la pression de ladite charge vaporisée soit égale à 0,695 MPa à l'issue de la compression.

Étape c)

La charge comprimée, est ensuite chauffée dans un échangeur E2 de type monophasique gaz, grâce à un échange de chaleur avec l'effluent issu du réacteur adiabatique de l'étape d). Dans ledit échangeur de type monophasique gaz, ladite charge comprimée est surchauffée à une température de 405 °C et l'effluent issu, à l'état gazeux, du dernier réacteur adiabatique de l'étape d) est "désurchauffé" sans être condensé et présente une température de 253 °C.

Ladite charge comprimée et chauffée dans ledit échangeur de type monophasique gaz est ensuite introduite dans un four de manière à l'amener à une température d'entrée dans le premier réacteur adiabatique de l'étape d) compatible avec la température de la réaction de déshydratation et de conversion du DEE en éthylène hautement endothermique, c'est-à-dire à une température de 440 °C.

Étape d)

Le piégeage des composés azotés dans l'étape a) de prétraitement permet de réduire significativement la température en entrée du premier réacteur adiabatique de l'étape d).

Ladite charge comprimée et chauffée est introduite dans le premier réacteur adiabatique à une pression d'entrée de 0,595 MPa. La pression de l'effluent en sortie du dernier réacteur adiabatique de l'étape e) est de 0,500 MPa. L'étape d) de déshydratation est opérée à une vitesse pondérale horaire de 7 h$^{-1}$.

Le réacteur adiabatique contient un lit fixe de catalyseur de déshydratation, ledit catalyseur comprenant 80 % poids de zéolithe ZSM-5 traitée avec $H_3PO_4$ de manière à ce que la teneur en phosphore P soit de 3%poids.

La conversion de la charge éthanol dans l'étape d) est de 95 %.
La température de sortie du dernier réacteur adiabatique de l'étape d) est de 420 °C.

Étape e)

L'effluent issu du dernier réacteur adiabatique de l'étape d) subit ensuite deux échanges de chaleur précédemment décrits et est envoyé dans la section de purification et recyclage.

Un effluent comprenant de l'éthylène aux spécifications finales est séparé Un effluent comprenant de l'eau correspondant a la purge du procédé est également séparé.
Un flux contenant les gaz légers et les impuretés est également séparés par une ou plusieurs distillation(s), éventuellement cryogénique(s),.
Un flux d'eau traitée en mélange avec une partie de l'éthanol non converti est recyclé en amont de l'étape b) de vaporisation dans les proportions décrites dans l'étape b).

Les différents flux, en kg/h, sont renseignés dans les Tableau 2 et Tableau 3.

Tableau 2 : Composition des principaux flux (1/2)

| Description du flux | | Charge éthanol prétraitée | Flux entrant dans l'étape (d) | Flux sortant de l'etape (d) | Effluent comprenant de l'éthylène |
|---|---|---|---|---|---|
| N°de flux correspondant sur la figure | | **2** | **7** | **8** | **12** |
| Débit massique total | kg/h | 45664 | 186916 | 186916 | 25692 |

(suite)

| Description du flux | | Charge éthanol prétraitée | Flux entrant dans l'étape (d) | Flux sortant de l'etape (d) | Effluent comprenant de l'éthylène |
|---|---|---|---|---|---|
| Débit massique par composants | kg/h | | | | |
| éthylène | | 0 | 0 | 25087 | 25087 |
| éthane | | 0 | 0 | 8 | 8 |
| C3 | | 0 | 0 | 93 | 93 |
| Description du flux | | Charge éthanol prétraitée | Flux entrant dans l'étape (d) | Flux sortant de l'etape (d) | Effluent comprenant de l'éthylène |
| C4 | | 0 | 0 | 87 | 87 |
| DEE | | 3352 | 3352 | 14 | 14 |
| éthanol | | 37504 | 39310 | 2187 | 151 |
| H$_2$0 | | 4808 | 143730 | 158602 | 198 |
| composés oxygénés (autres que éthanol) | | 0 | 325 | 586 | 42 |
| Autres composants minoritaires | | 0 | 199 | 252 | 12 |

Tableau 3 : Composition des principaux flux (2/2)

| Description du flux | | Effluent comprenant de l'eau | Recycle d'éthanol et d'eau | Eau purgée | gaz légers |
|---|---|---|---|---|---|
| N° de flux correspondant sur la figure | | **13** | **14** | **13** | **11** |
| Débit massique total | kg/h | 161224 | 141252 | 19007 | 965 |
| Débit massique par composants | kg/h | | | | |
| éthylène | | 0 | 0 | 0 | 0 |
| éthane | | 0 | 0 | 0 | 0 |
| C3 | | 0 | 0 | 0 | 0 |
| C4 | | 0 | 0 | 0 | 0 |
| DEE | | 0 | 0 | 0 | 0 |
| éthanol | | 2036 | 1806 | 3 | 227 |
| H$_2$0 | | 158404 | 138922 | 18987 | 495 |
| composés oxygénés (autres que éthanol) | | 544 | 325 | 6 | 213 |
| Autres composants minoritaires | | 240 | 199 | 11 | 30 |

Les composés C3 et C4 sont des composés hydrocarbonés en C3 et C4.

La sélectivité du procédé en éthylène est de 99 %.

Elle est calculée de la façon suivante : (Éthylène contenu dans l'effluent comprenant de l'éthylène) / (0.61 *quantité d'éthanol converti) où la quantité d'éthanol converti est l'éthanol contenu dans la charge éthanol avant prétraitement soustrait de l'éthanol contenu dans les flux d'eau purgée et dans l'effluent comprenant de l'éthylène. 0,61 g est la quantité maximale d'éthylène obtenue en déshydratant 1 g d'éthanol pur.

Le bilan énergétique du schéma selon l'exemple 1 conforme à l'invention est renseigné dans le Tableau 4:

Tableau 4 : bilan énergétique

| Énergie échangée à l'intérieur du système | | Énergie fournie au système par un apport externe | | |
|---|---|---|---|---|
| Quantité de chaleur échangée dans le premier échangeur (E1) | Quantité de chaleur échangée dans le second échangeur (E2) | Quantité de chaleur échangée dans le four | Puissance nécessaire à la compression | Quantité de chaleur extraite sur la colonne de séparation gaz/ liquide |
| MW | MW | MW | MW | MW |
| 93,6 | 18,32 | 10,4 | 10,9 | 22,53 |

L'estimation de la consommation en énergie primaire a été réalisée en utilisant les bases suivantes :

- efficacité de 0,8 sur les fours

- efficacité de 0,375 sur la production d'électricité

Le schéma selon l'exemple 1 conforme à l'invention présente une consommation en énergie primaire équivalente ou consommation spécifique de 6,0 GJ équivalent par tonne d'éthylène produit.

**Exemple 2 : comparatif**

L'exemple 2 illustre un procédé dans lequel l'étape de prétraitement n'a pas lieu. L'éthanol n'est pas converti en DEE et le procédé démarre à l'étape b).

Étape b)

La charge de vaporisation, constituée de la charge éthanol non prétraitée en mélange avec 141 258 kg/h d'eau traitée et d'éthanol non converti recyclés selon l'étape e), est introduite, à un débit de 186 922 kg/h dans l'échangeur E1 à une pression égale à 0,24 MPa et à une température de 120°C.

Par rapport à l'exemple 1, la pression a été abaissée de 0,03 MPa. Sans présence de DEE, la température de bulle de la charge de vaporisation à 0,27 MPa est de 115 °C (127°C dans l'exemple 1). La pression d'entrée est modifiée de 0,03 MPa de manière à conserver une approche thermique minimale de 15 °C avec l'effluent issu du dernier réacteur adiabatique de l'étape d).

Dans l'étape c), la majorité de la chaleur latente de la phase aqueuse de l'effluent issu du réacteur adiabatique de l'étape d) est récupérée pour vaporiser la charge de vaporisation, sans apport de chaleur externe. Ainsi, 98 MW sont échangés entre la charge de vaporisation et l'effluent du réacteur.

Étape optionnelle de compression

La charge vaporisée est ensuite comprimée dans l'étape optionnelle de compression grâce à un compresseur radial à multiplicateur intégré de manière à ce que la pression de ladite charge vaporisée à l'issue de la compression soit égale à 0,695 MPa.

Étape c)

La charge comprimée est ensuite chauffée dans un échangeur E2 de type monophasique gaz, grâce à un échange de chaleur avec l'effluent issu du dernier réacteur adiabatique de l'étape d). Dans ledit échangeur de type monophasique gaz, ladite charge comprimée est surchauffée à une température de 405 °C et l'effluent issu, à l'état gazeux, du dernier réacteur adiabatique de l'étape d) est "désurchauffé" sans être condensé et présente une température de 269 °C.

Étape d)

Ladite charge comprimée et chauffée dans ledit échangeur de type monophasique gaz est ensuite introduite dans un four de manière à l'amener à une température d'entrée dans le premier réacteur adiabatique de l'étape d) compatible avec la température de la réaction de déshydratation, c'est-à-dire à une température de 470 °C. La température de sortie du dernier réacteur adiabatique de l'étape d) est de 420 °C.

Ladite charge comprimée et chauffée est introduite dans le réacteur adiabatique à une pression d'entrée de 0,595 MPa. La pression de l'effluent en sortie du dernier réacteur adiabatique de l'étape d) est de 0,500 MPa. L'étape d) de déshydratation est opérée à une vitesse pondérale horaire de 7 h⁻¹.

La conversion de la charge éthanol dans l'étape d) est de 95 %.

Étape e)

L'effluent issu du dernier réacteur adiabatique de l'étape d) subit ensuite les deux échanges de chaleur précédemment décrits et est envoyé dans la section de purification et recyclage.

Un effluent comprenant de l'éthylène aux spécifications finales est séparé Un effluent comprenant de l'eau correspondant a la purge du procédé est également séparé.

Un flux contenant les gaz légers et les impuretés est également séparés par une ou plusieurs distillation(s), éventuellement cryogénique(s),.

Un flux d'eau traitée en mélange avec une partie de l'éthanol non converti est recyclé en amont de l'étape b) de vaporisation dans les proportions décrites dans l'étape b).

Les différents flux, en kg/h, sont renseignés dans les Tableau 5 et Tableau 6.

Tableau 5 : Composition des principaux flux (1/2)

| Description du flux | | Charge éthanol | Flux entrant dans R1 | Flux sortant de R2 | Effluent comprenant de l'éthylène |
|---|---|---|---|---|---|
| N°de flux correspondant sur la figure | | **2** | **7** | **8** | **12** |
| Débit massique total | kg/h | 45664 | 186922 | 186922 | 25964 |
| **Débit massique par composants** | **kg/h** | | | | |
| éthylène | | 0 | 0 | 25087 | 25087 |
| éthane | | 0 | 0 | 8 | 8 |
| C3 | | 0 | 0 | 93 | 93 |
| C4 | | 0 | 0 | 87 | 87 |
| DEE | | 0 | 0 | 14 | 14 |
| éthanol | | 41671 | 43496 | 2187 | 151 |
| $H_2O$ | | 3993 | 142947 | 158602 | 311 |
| composés oxygénés (autres que éthanol) | | 0 | 413 | 586 | 62 |
| Autres composants minoritaires | | 0 | 66 | 258 | 151 |

Tableau 6 : Composition des principaux flux (1/2)

| Description du flux | | Effluent comprenant de l'eau | Recycle d'éthanol et d'eau | Eau purgée | gaz légers |
|---|---|---|---|---|---|
| N° de flux correspondant sur la figure | | **13** | **14** | **13** | **11** |
| Débit massique total | kg/h | 160958 | 141258 | 19007 | 693 |

(suite)

| Description du flux | | Effluent comprenant de l'eau | Recycle d'éthanol et d'eau | Eau purgée | gaz légers |
|---|---|---|---|---|---|
| **Débit massique par composants** | **kg/h** | | | | |
| éthylène | | 0 | 0 | 0 | 0 |
| éthane | | 0 | 0 | 0 | 0 |
| C3 | | 0 | 0 | 0 | 0 |
| C4 | | 0 | 0 | 0 | 0 |
| DEE | | 0 | 0 | 0 | 0 |
| éthanol | | 2036 | 1825 | 3 | 208 |
| $H_2O$ | | 158291 | 138954 | 18987 | 350 |
| composés oxygénés (autres que éthanol) | | 524 | 413 | 6 | 105 |
| Autres composants minoritaires | | 107 | 66 | 11 | 30 |

Les composés C3 et C4 sont des composés hydrocarbonés en C3 et C4.
La sélectivité du procédé en éthylène est de 99 %.

Le bilan énergétique du schéma selon l'exemple 2 est renseigné dans le Tableau 7.

Tableau 7 : Bilan énergétique

| Énergie échangée à l'intérieur du système | | Énergie fournie au système par un apport externe | | |
|---|---|---|---|---|
| Quantité de chaleur échangée dans le premier échangeur(E1) | Quantité de chaleur échangée dans le second échangeur(E2) | Quantité de chaleur échangée dans le four | Électricité requise pour la compression | Quantité de chaleur extraite sur la colonne de séparation gaz/ liquide |
| MW | MW | MW | MW | MW |
| 93,8 | 17,1 | 13,9 | 12,4 | 26,7 |

Le schéma selon l'exemple 2 comparatif à l'invention présente une consommation en énergie primaire équivalente ou consommation spécifique de 7,23 GJ équivalent par tonne d'éthylène produit.
Sans prétraitement, la consommation en énergie primaire augmente donc de 1,2 GJ équivalent par tonne d'éthylène produit.

**Revendications**

1. Procédé de déshydratation d'une charge éthanol en éthylène comprenant au moins les étapes :

   a) Une étape de prétraitement de la charge éthanol sur un solide acide opérant à une température comprise entre 100 et 130°C de manière à produire une charge éthanol prétraitée,
   b) Une étape de vaporisation d'une charge de vaporisation comprenant ladite charge éthanol prétraitée dans un échangeur de chaleur, ladite charge de vaporisation étant introduite dans ladite étape de vaporisation à une pression comprise entre 0,1 et 2,5 MPa de manière à produire une charge vaporisée,
   c) Une étape de surchauffe de ladite charge vaporisée de manière à l'amener à une température d'entrée compatible avec la température de la réaction de déshydratation c'est à dire à une température comprise entre 350 et 550 °C,
   d) Une étape de déshydratation de ladite charge issue de l'étape c) dans au moins un réacteur adiabatique contenant au moins un catalyseur de déshydratation et dans lequel la réaction de déshydratation a lieu, opérant à une température d'entrée comprise entre 350 et 550 °C et à une pression d'entrée comprise entre 0,3 et 1,8 MPa,

**2.** Procédé selon la revendication 1 dans lequel est ladite charge éthanol est une charge éthanol produite à partir de source renouvelable issue de la biomasse.

**3.** Procédé selon l'une des revendications 1 à 2 dans lequel ladite charge de vaporisation est introduite dans ladite étape b) de vaporisation à une pression comprise entre 0,1 et 1,4 MPa et comprenant une étape de compression de ladite charge vaporisée préalablement à ladite étape c) de surchauffe.

**4.** Procédé selon la revendication 3 dans lequel la pression de la charge comprimée est comprise entre 0,3 et 1,8 MPa.

**5.** Procédé selon l'une des revendications 1 à 4 dans lequel ladite charge vaporisée, éventuellement comprimée, est chauffée dans un échangeur de type monophasique gaz, grâce à un échange de chaleur avec l'effluent issu du dernier réacteur adiabatique de l'étape d).

**6.** Procédé selon l'une des revendications 1 à 5 dans lequel l'effluent issu du dernier réacteur adiabatique de l'étape d) présente en sortie du dernier réacteur adiabatique de l'étape d) une température comprise entre 270 et 450°C.

**7.** Procédé selon l'une des revendications 1 à 6 dans lequel l'effluent issu du dernier réacteur adiabatique de l'étape d) présente en sortie du dernier réacteur adiabatique de l'étape d) une pression comprise entre 0,2 et 1,6 MPa.

**8.** Procédé selon l'une des revendications 1 à 7 dans lequel l'étape d) de déshydratation est réalisée dans un ou deux réacteurs.

**9.** Procédé selon l'une des revendications 1 à 8 dans lequel ledit catalyseur de déshydratation utilisé dans l'étape d) est un catalyseur acide amorphe ou un catalyseur acide zéolithique.

**10.** Procédé selon l'une des revendications 1 à 9 dans lequel ladite charge éthanol est une charge éthanol concentrée, c'est à dire une charge éthanol comprenant un pourcentage massique en éthanol supérieur ou égal à 35% poids.

**11.** Procédé selon la revendication 10 dans lequel ladite charge éthanol concentrée comprend un pourcentage massique en éthanol compris entre 35 et 99,9% poids.

**12.** Procédé selon l'une des revendications 1 à 11 dans lequel l'étape a) de prétraitement est complétée par un prétraitement utilisant une résine échangeuse d'anion.

**Patentansprüche**

**1.** Verfahren zur Dehydrierung einer Ethanolcharge zu Ethylen, umfassend mindestens die folgenden Schritte:

a) einen Schritt der Vorbehandlung der Ethanolcharge auf einem sauren Feststoff, der bei einer Temperatur zwischen 100 und 130 °C wirkt, um eine vorbehandelte Ethanolcharge zu erzeugen,
b) einen Schritt der Verdampfung einer Verdampfungscharge, umfassend die vorbehandelte Ethanolcharge, in einem Wärmetauscher, wobei die Verdampfungscharge in den Verdampfungsschritt bei einem Druck zwischen 0,1 und 2,5 MPa eingeleitet wird, um eine verdampfte Charge zu erzeugen,
c) einen Schritt der Überhitzung der verdampften Charge, um sie auf eine Eingangstemperatur zu bringen, die mit der Temperatur der Dehydrierungsreaktion kompatibel ist, d.h. auf eine Temperatur zwischen 350 und 550 °C,
d) einen Schritt der Dehydrierung der aus Schritt c) stammenden Charge in mindestens einem adiabatischen Reaktor, der mindestens einen Dehydrierungskatalysator enthält, und in dem die Dehydrierungsreaktion stattfindet, der bei einer Eingangstemperatur zwischen 350 und 550 °C und einem Eingangsdruck zwischen 0,3 und 1,8 MPa wirkt.

**2.** Verfahren nach Anspruch 1, bei dem die Ethanolcharge eine Ethanolcharge ist, die aus einer von Biomasse stammenden erneuerbaren Quelle erzeugt wird.

**3.** Verfahren nach einem der Ansprüche 1 bis 2, bei dem die Verdampfungscharge in dem Verdampfungsschritt b) bei einem Druck zwischen 0,1 und 1,4 MPa eingeleitet wird, und umfassend einen Schritt der Kompression der verdampften Charge vor dem Überhitzungsschritt c).

4. Verfahren nach Anspruch 3, bei dem der Druck der komprimierten Charge zwischen 0,3 und 1,8 MPa beträgt.

5. Verfahren nach einem der Ansprüche 1 bis 4, bei dem die verdampfte Charge, die eventuell komprimiert ist, in einem Gastauscher monophasischen Typs dank des Wärmeaustauschs mit dem vom letzten adiabatischen Reaktor aus Schritt d) kommenden Abwasser erhitzt wird.

6. Verfahren nach einem der Ansprüche 1 bis 5, bei dem der aus dem letzten adiabatischen Reaktor aus Schritt d) kommende Abstrom am Ausgang des letzten adiabatischen Reaktors aus Schritt d) eine Temperatur zwischen 270 und 450 °C aufweist.

7. Verfahren nach einem der Ansprüche 1 bis 6, bei dem der vom letzten adiabatischen Reaktor aus Schritt d) kommende Abstrom am Ausgang des letzten adiabatischen Reaktors aus Schritt d) einen Druck zwischen 0,2 und 1,6 MPa aufweist.

8. Verfahren nach einem der Ansprüche 1 bis 7, bei dem der Schritt d) der Dehydrierung in einem oder zwei Reaktoren durchgeführt wird.

9. Verfahren nach einem der Ansprüche 1 bis 8, bei dem der Dehydrierungskatalysator, der in Schritt d) verwendet wird, ein saurer amorpher Katalysator oder ein saurer zeolithischer Katalysator ist.

10. Verfahren nach einem der Ansprüche 1 bis 9, bei dem die Ethanolcharge eine konzentrierte Ethanolcharge ist, d.h. eine Ethanolcharge, umfassend einen Massenprozentsatz an Ethanol größer oder gleich 35 Gew.-%.

11. Verfahren nach Anspruch 10, bei dem die konzentrierte Ethanolcharge einen Massenprozentsatz an Ethanol zwischen 35 und 99,9 Gew.-% umfasst.

12. Verfahren nach einem der Ansprüche 1 bis 11, bei dem der Schritt a) der Vorbehandlung durch eine Vorbehandlung, die ein Anionentauscharz verwendet, ergänzt wird.

**Claims**

1. Process for dehydration of an ethanol feedstock into ethylene comprising at least the stages:

   a) A stage for pretreatment of the ethanol feedstock on an acidic solid operating at a temperature of between 100 and 130°C in such a way as to produce a pretreated ethanol feedstock,
   b) A stage for evaporation of an evaporation feedstock comprising said ethanol feedstock that is pretreated in a heat exchanger, with said evaporation feedstock being introduced into said evaporation stage at a pressure of between 0.1 and 2.5 MPa in such a way as to produce an evaporated feedstock,
   c) A stage for superheating said evaporated feedstock in such a way as to bring it to an inlet temperature that is compatible with the temperature of the dehydration reaction, i.e., to a temperature of between 350 and 550°C,
   d) A stage for dehydration of said feedstock that is obtained from stage c) in at least one adiabatic reactor that contains at least one dehydration catalyst and in which the dehydration reaction takes place, operating at an inlet temperature of between 350 and 550°C and at an inlet pressure of between 0.3 and 1.8 MPa.

2. Process according to Claim 1, in which said ethanol feedstock is an ethanol feedstock that is produced from a renewable source that is obtained from biomass.

3. Process according to one of Claims 1 to 2, in which said evaporation feedstock is introduced into said evaporation stage b) at a pressure of between 0.1 and 1.4 MPa and comprising a compression stage of said feedstock that is evaporated prior to said superheating stage c).

4. Process according to Claim 3, in which the pressure of the compressed feedstock is between 0.3 and 1.8 MPa.

5. Process according to one of Claims 1 to 4, in which said evaporated feedstock, optionally compressed, is heated in an exchanger of the gas single-phase type, using a heat exchange with the effluent that is obtained from the last adiabatic reactor of stage d).

6. Process according to one of Claims 1 to 5, in which the effluent that is obtained from the last adiabatic reactor of stage d) has - exiting the last adiabatic reactor of stage d) - a temperature of between 270 and 450°C.

7. Process according to one of Claims 1 to 6, in which the effluent that is obtained from the last adiabatic reactor of stage d) has - exiting from the last adiabatic reactor of stage d) - a pressure of between 0.2 and 1.6 MPa.

8. Process according to one of Claims 1 to 7, in which the dehydration stage d) is carried out in one or two reactors.

9. Process according to one of Claims 1 to 8, in which said dehydration catalyst that is used in stage d) is an amorphous acid catalyst or a zeolitic acid catalyst.

10. Process according to one of Claims 1 to 9, in which said ethanol feedstock is a concentrated ethanol feedstock, i.e., an ethanol feedstock that comprises a percent by mass of ethanol that is greater than or equal to 35% by weight.

11. Process according to Claim 10, in which said concentrated ethanol feedstock comprises a percent by mass of ethanol that is between 35 and 99.9% by weight.

12. Process according to one of Claims 1 to 11, in which the pretreatment stage a) is completed by a pretreatment using an anion exchange resin.

## RÉFÉRENCES CITÉES DANS LA DESCRIPTION

**Documents brevets cités dans la description**

- US 4232179 A
- WO 2007134415 A
- US 4396789 A
- WO 2011002699 A
- WO 2010060981 A
- JP 11043452 A
- WO 2009098262 A
- WO 2009098267 A
- WO 2009098268 A
- WO 2009098269 A

**Littérature non-brevet citée dans la description**

- **H. KNÖZINGER ; R. KÖHNE.** The Deshydration of Alcohols over Alumina. I:The reaction scheme. *Journal of Catalysis,* 1966, vol. 5, 264-270
- **S.N. CHAUDHURI.** Reactions of ethanol over ZSM-5. *Journal of Molecular Catalysis,* 1990, vol. 62, 289-295
- **DANIEL BALLERINI.** Les Biocarburants, État des lieux, perspectives et enjeux du développement